# EUROPÄISCHE PATENTSCHRIFT

(11) **EP 3 587 395 B1**
(45) Veröffentlichungstag und Bekanntmachung des Hinweises auf die Patenterteilung: **09.08.2023**
(21) Anmeldenummer: 19180422.8
(22) Anmeldetag: 17.06.2019
(51) Int. Cl.: C07C 279/18, C08J 3/24, C08K 5/31, B60C 1/00

(54) **MIT ETHYLENISCH UNGESÄTTIGTEN GRUPPEN MODIFIZIERTES DIPHENYLGUANIDIN**
DIPHENYLGUANIDINE MODIFIED WITH ETHYLENICALLY UNSATURATED GROUPS
DIPHÉNYLGUANIDINE MODIFIÉE DE GROUPES ÉTHYLÉNIQUEMENT INSATURÉS

(30) Priorität: 21.06.2018 DE 102018210095
(43) Veröffentlichungstag der Anmeldung: 01.01.2020
(73) Patentinhaber: Continental Reifen Deutschland GmbH, 30165 Hannover (DE)
(72) Erfinder: Jacob, Andreas, 30165 Hannover (DE); Kendziorra, Norbert, 30165 Hannover (DE); Dauer, David-Raphael, 30165 Hannover (DE); Strohmeier, Julian, 30165 Hannover (DE)
(74) Vertreter: Continental Corporation

(56) Entgegenhaltungen:
- WO-A1-2016/030469
- DE-A1-102015 214 731
- US-A1- 2003 135 006
- SCHERZ M W ET AL: "SYNTHESIS AND STRUCTURE-ACTIVITY RELATIONSHIPS ON N,N'DI-O- TOLYGUANIDINE ANALOGUES, HIGH-AFFINITY LIGANDS FOR THE HALOPERIDOL-SENSITIVE ALPHA RECEPTOR", JOURNAL OF MEDICINAL CHEMISTRY, AMERICAN CHEMICAL SOCIETY, US, Bd. 33, Nr. 9, 1. Januar 1990 (1990-01-01) , Seiten 2421-2429, XP000652226, ISSN: 0022-2623, DOI: 10.1021/JM00171A016

## Beschreibung

Die vorliegende Erfindung betrifft ein mit ethylenisch ungesättigten Gruppen modifiziertes Diphenylguanidin, eine dieses modifizierte Diphenylguanidin umfassende Kautschukmischung, ein Verfahren zur Herstellung eines Kautschukvulkanisats aus dieser Kautschukmischung sowie das entsprechende Kautschukvulkanisat, und ein dieses Vulkanisat enthaltendes Kautschukprodukt. Die vorliegende Erfindung betrifft ferner die Verwendung des modifizierten Diphenylguanidins als Sekundärbeschleuniger bei der Vulkanisation von Kautschukmischungen.

Der Gegenstand der Erfindung ist in den beigefügten Patentansprüchen definiert.

Das Verfahren der Vulkanisation ist bei der Herstellung von Kautschukprodukten wie beispielsweise Gummiartikeln oder Reifen von zentraler Bedeutung. Es ist seit Langem bekannt, dass der Vulkanisationsvorgang durch Zugabe basischer organischer Verbindungen beschleunigt werden kann. Dazu wurden zunächst beispielsweise Piperidin, Anilin und andere stickstoffhaltige organische Verbindungen wie Hexamethylentetramin oder Thiocarbanilid als Beschleuniger vorgeschlagen. Heute kommen hingegen vor allem Beschleuniger auf Schwefelbasis zum Einsatz. Diese bieten generell den Vorteil, dass durch die Verwendung unterschiedlicher Beschleuniger und deren Kombinationen die Produkt- und Verarbeitungseigenschaften (insbesondere die Induktionsperiode sowie die Reaktionsgeschwindigkeit bei der Vulkanisation) über einen weiten Bereich eingestellt werden können.

Zur weiteren Regulierung der Induktions- und Vulkanisationszeit können den Kautschukmischungen sogenannte Zweitbeschleuniger zugesetzt werden. Am weitesten verbreitet sind dabei die Guanidin-Beschleuniger und insbesondere Diphenylguanidin (DPG). Diese erlauben nicht nur eine Einstellung des Vulkanisationsverhaltens, sondern verbessern ebenfalls weitere relevante Stoffeigenschaften der Kautschukmischung (insbesondere die Mooney-Viskosität) sowie des daraus erhaltenen Vulkanisats (insbesondere die Bruchdehnung und die Zugfestigkeit). Gerade füllstoffhaltige Kautschukmischungen, also Kautschukmischungen, die beispielsweise einen Füllstoff auf Basis von Kieselsäure bzw. Silica oder Ruß enthalten und die für Reifen eingesetzt werden, weisen hohe Mooney-Viskositäten auf, was die Verarbeitung deutlich erschwert. Durch die Zugabe von Zweitbeschleunigern auf Guanidin-Basis zu diesen Kautschukmischungen kann die Mooney-Viskosität herabgesetzt und so die Verarbeitung erleichtert werden.

Der Einsatz von Guanidin-Beschleunigern in schwefelvernetzbaren Kautschukmischungen ist beispielsweise in der deutschen Patentanmeldung DE 102015214731 A1 offenbart. Guanidin-Verbindungen sind auch aus anderen Technikbereichen bekannt. Der Artikel M. W. Scherz et al., J. Med. Chem. 1990, 33, 2421-2429 offenbart insoweit eine Studie zur Struktur-Affinitäts-Beziehung verschiedener Guanidin-Verbindungen mit einer Diphenylguanidin-Grundstruktur aus dem Bereich der medizinischen Forschung.

Die Guanidin-Beschleuniger haben aber den generellen Nachteil, dass bei der Vulkanisation leichtflüchtige organische Aminverbindungen freigesetzt werden; bei der Verwendung des am weitesten verbreiteten Guanidin-Beschleunigers DPG wird beispielsweise Anilin freigesetzt. Da aber DPG als toxisch gilt und Kontaktdermatitis hervorrufen kann, und Anilin als kanzerogen gilt, besteht das allgemeine Bestreben, auf DPG als Zweitbeschleuniger zu verzichten.

Daher wurde beispielsweise in der internationalen Patentanmeldung WO 2016/030469 vorgeschlagen, DPG in Kautschukmischungen, die einen Kieselsäure-basierten Füllstoff und/oder Ruß enthalten, durch Polyethylenimin zu ersetzen. In G. Heinrich et al., KGK Rubberpoint 2013, 1-2, 25 wird der Einsatz der natürlich vorkommenden Aminosäure L-Cystin und deren Derivat L-Cystin-Dimethylesterdihydrochlroid als umweltfreundliche Zweitbeschleuniger bei der Vulkanisation von Silica-gefüllten Lösungs-Styrol-Butadien-Kautschuken beschrieben.

Diese Ersatzstoffe für DPG weisen aber den Nachteil auf, dass sie chemisch völlig verschieden von DPG sind. Wenn diese großtechnisch als Ersatz für DPG eingesetzt werden sollen, erfordert dies daher eine grundlegende Anpassung des zuvor mit DPG durchgeführten Vulkanisationsverfahrens.

Der vorliegenden Erfindung liegt somit die Aufgabe zugrunde, einen Ersatz für Diphenylguanidin (DPG) bereitzustellen, bei dessen Einsatz als Zweitbeschleuniger in der Vulkanisation die Freisetzung von Anilin verringert oder vermieden wird und gleichzeitig keine grundlegende Anpassung des Vulkanisationsverfahrens notwendig ist.

Diese Aufgabe wird durch die in den Ansprüchen gekennzeichneten Ausführungsformen gelöst, d.h. insbesondere durch eine erfindungsgemäß Verbindung mit der allgemeinen Formel (I) 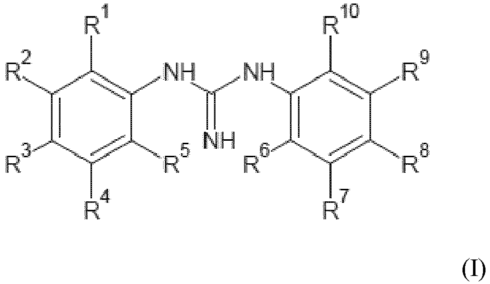
wobei die Reste R¹ bis R¹⁰ unabhängig voneinander aus der Gruppe, bestehend aus Wasserstoff, und -Y-C(R¹²)=C(R¹³)(R¹⁴) ausgewählt sind,
wobei
R¹², R¹³ und R¹⁴ jeweils unabhängig voneinander Wasserstoff, Halogen, geradkettiges oder verzweigtes C₁-C₁₂-Alkyl, C₃-C₈-Cycloalkyl, C₆-C₁₂-Aryl oder C₅-C₁₂-Heteroaryl ist,
wobei das geradkettige oder verzweigte C₁-C₁₂-Alkyl, C₃-C₈-Cycloalkyl, C₆-C₁₂-Aryl und C₅-C₁₂-Heteroaryl optional mit einem oder mehreren Substituenten, ausgewählt aus der Gruppe, bestehend aus Halogen, Hydroxyl, Cyano, Nitro, Aryl und Benzyl, substituiert sein kann,
dadurch gekennzeichnet, dass einer der Reste R¹ bis R⁵ sowie einer der Reste R⁶ bis R¹⁰ -Y-C(R¹²)=C(R¹³)(R¹⁴) ist, dabei Y jeweils eine Einfachbindung ist und die verbleibenden Reste von R¹ bis R¹⁰ Wasserstoff sind.

Nicht Teil der Erfindung, jedoch allgemeiner offenbart ist eine Verbindung mit der allgemeinen Formel (I) 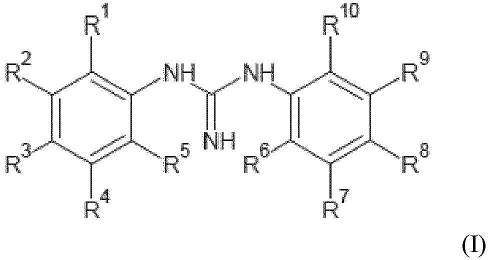
wobei die Reste R¹ bis R¹⁰ unabhängig voneinander aus der Gruppe, bestehend aus Wasserstoff, Halogen, -X-S-C(=O)(R¹¹), -Y-C(R¹²)=C(R¹³)(R¹⁴) und -Z-Si(OR¹⁵)ₙ(R¹⁶)₃₋ₙ, ausgewählt sind,
mit der Maßgabe, dass mindestens einer der Reste R¹ bis R¹⁰ -Y-C(R¹²)=C(R¹³)(R¹⁴) ist, wobei
X, Y und Z jeweils unabhängig voneinander eine Einfachbindung, C₁-C₆-Alkylen, C₂-C₆-Alkenylen oder C₂-C₆-Alkinylen ist,
R¹¹ geradkettiges oder verzweigtes C₁-C₁₂-Alkyl, C₃-C₈-Cycloalkyl, C₆-C₁₂-Aryl oder C₅-C₁₂-Heteroaryl ist, welche optional mit einem oder mehreren Substituenten, ausgewählt aus der Gruppe, bestehend aus Halogen, Hydroxyl, Cyano, Nitro, Aryl und Benzyl, substituiert sein können,
R¹², R¹³ und R¹⁴ jeweils unabhängig voneinander Wasserstoff, Halogen, geradkettiges oder verzweigtes C₁-C₁₂-Alkyl, C₃-C₈-Cycloalkyl, C₆-C₁₂-Aryl oder C₅-C₁₂-Heteroaryl ist, wobei das geradkettige oder verzweigte C₁-C₁₂-Alkyl, C₃-C₈-Cycloalkyl, C₆-C₁₂-Aryl und C₅-C₁₂-Heteroaryl optional mit einem oder mehreren Substituenten, ausgewählt aus der Gruppe, bestehend aus Halogen, Hydroxyl, Cyano, Nitro, Aryl und Benzyl, substituiert sein kann,
R¹⁵ und R¹⁶ jeweils unabhängig voneinander geradkettiges oder verzweigtes C₁-C₁₂-Alkyl, C₃-C₈-Cycloalkyl, C₆-C₁₂-Aryl oder C₅-C₁₂-Heteroaryl ist, welche optional mit einem oder mehreren Substituenten, ausgewählt aus der Gruppe, bestehend aus Halogen, Cyano, Nitro, Aryl und Benzyl, substituiert sein können, und
n eine ganze Zahl von 1 bis 3 ist.

Bei der offenbarten Verbindung gemäß der allgemeinen Formel (I) handelt es sich um ein Diphenylguanidin, welches mit zumindest einer ethylenisch ungesättigten Gruppe modifiziert ist. Daher wird die offenbarte Verbindung nachfolgend auch als modifiziertes Diphenylguanidin bezeichnet, in Abgrenzung zum aus dem Stand der Technik bekannten unmodifizierten Diphenylguanidin (DPG), bei dem R¹R¹⁰ in der allgemeinen Formel (I) jeweils Wasserstoff ist.

Die offenbarten Verbindungen haben den Vorteil, dass diese bei Verwendung im Vulkanisationsprozess keine nennenswerten Mengen an Anilin freisetzen. Die Spaltprodukte enthalten nämlich zumindest einen ethylenisch ungesättigten Rest, der während der Vulkanisation abreagieren und mit dem Polymer vernetzen kann. Auf diese Weise werden die Spaltprodukte bei der Vulkanisation in das Netzwerk des Vulkanisats eingebunden, so dass es zu keiner bzw. zu einer verringerten Freisetzung potentiell gesundheits- und umweltschädlicher Spaltprodukte während und nach der Vulkanisation kommt.

Das modifizierte Guanidin gemäß der allgemeinen Formel (I) weist die Reste R¹ bis R¹⁰ auf. Diese sind unabhängig voneinander aus der Gruppe, bestehend aus Wasserstoff, Halogen, -X-S-C(=O)(R¹¹), -Y-C(R¹²)=C(R¹³)(R¹⁴) und -Z-Si(OR¹⁵)ₙ(R¹⁶)₃₋ₙ, ausgewählt. Erfindungsgemäß sind die Reste R¹ bis R¹⁰ unabhängig voneinander aus Wasserstoff und -Y-C(R¹²)=C(R¹³)(R¹⁴) ausgewählt. Halogen kann dabei Fluor, Chlor, Brom oder Iod sein. Vorzugsweise handelt es sich bei dem Halogen um Fluor oder Chlor. Um die vorstehend beschriebenen Vorteile der offenbarten Verbindungen zu erzielen, ist zumindest einer der Reste R¹ bis R¹⁰ ein ethylenisch ungesättigter Rest -Y-C(R¹²)=C(R¹³)(R¹⁴). Weitere Reste können weitere ethylenisch ungesättigte Reste -Y-C(R¹²)=C(R¹³)(R¹⁴), schwefelhaltige Reste -X-S-C(=O)(R¹¹) oder silanhaltige Reste mit kondensationsfähigen Gruppen -Z-Si(OR¹⁵)ₙ(R¹⁶)₃₋ₙ mit n = 1 bis 3 sein. Über die schwefelhaltigen Reste können die Spaltprodukte der offenbarten Verbindungen ebenfalls während der Vulkanisation in das Netzwerk des Vulkanisats eingebunden werden. Die silanhaltigen Reste enthalten mindestens eine kondensationsfähige Gruppe OR¹⁵, die nach Hydrolyse an gegebenenfalls in der zu vulkanisierenden Kautschukmischung vorhandenem Silica anbinden und auf diese Weise die Spaltprodukte der offenbarten Verbindungen in das Netzwerk des Vulkanisats einbinden kann.

Enthält die offenbarte Verbindung mehrere ethylenisch ungesättigte Reste -Y-C(R¹²)=C(R¹³)(R¹⁴), können diese Reste jeweils gleich oder verschieden sein, d.h. Y, R¹², R¹³ und R¹⁴ können jeweils gleich oder verschieden sein. Gleiches gilt für die weiteren möglichen Reste Halogen, die schwefelhaltigen Reste sowie die silanhaltigen Reste mit kondensationsfähigen Gruppen.

X, Y und Z sind jeweils unabhängig voneinander eine Einfachbindung, C₁-C₆-Alkylen, C₂-C₆-Alkenylen oder C₂-C₆-Alkinylen. C₂-C₆-Alkylen ist eine verbrückende Gruppe und umfasst -(CH₂)ₘ- mit m = 1 bis 6, aber auch entsprechende verzweigte Gruppen. Bevorzugtes C₁-C₆-Alkylen ist -(CH₂)- (Methylen) und -(CH₂CH₂)- (Ethylen). C₂-C₆-Alkenylen ist eine verbrückende Gruppe mit mindestens einer Doppelbindung und umfasst beispielsweise -CH=CH-(CH₂)ₚ- mit p = 0 bis 4, aber auch entsprechende verzweigte und/oder mehrfach ungesättigte Gruppen. Bevorzugtes C₂-C₆-Alkenylen ist -CH=CH-. C₂-C₆-Alkinylen ist eine verbrückende Gruppe mit mindestens einer Dreifachbindung und umfasst beispielsweise -C=C-(CH₂)_{q}- mit q = 0 bis 4, aber auch entsprechende verzweigte und/oder mehrfach ungesättigte Gruppen. Bevorzugtes C₂-C₆-Alkinylen ist -C≡C-. Gemäß einer bevorzugten Ausführungsform der offenbarten Verbindungen sind X, Y und Z eine Einfachbindung, -(CH₂)- (Methylen) oder -(CH₂CH₂)- (Ethylen).

Der Rest R¹¹ ist geradkettiges oder verzweigtes C₁-C₁₂-Alkyl, C₃-C₈-Cycloalkyl, C₆-C₁₂-Aryl oder C₅-C₁₂-Heteroaryl. C₁-C₁₂-Alkyl ist beispielsweise Methyl, Ethyl, Propyl, iso-Propyl, Butyl, iso-Butyl, tert-Butyl, Pentyl, Hexyl, Heptyl, Octyl, Nonyl, Decyl, Undecyl oder Dodecyl. Vorzugsweise ist C₁-C₁₂-Alkyl Methyl, Ethyl, Propyl, iso-Propyl, Butyl, Pentyl, Hexyl, Heptyl oder Octyl. C₃-C₈-Cycloalkyl ist beispielsweise Cyclopropyl, Cyclobutyl, Cyclopentyl, Cyclohexyl, Cycloheptyl oder Cyclooctyl. Vorzugsweise ist C₃-C₈-Cycloalkyl Cyclohexyl. C₆-C₁₂-Aryl oder C₅-C₁₂-Heteroaryl sind beispielsweise Phenyl, Naphthyl, Furanyl, Imidazolyl, Pyrazolyl oder Pyridinyl. Vorzugsweise sind C₆-C₁₂-Aryl oder C₅-C₁₂-Heteroaryl Phenyl, Imidazolyl oder Pyridinyl. Die vorstehend genannten Substituenten können optional mit einem oder mehreren Substituenten, ausgewählt aus der Gruppe, bestehend aus Halogen, Hydroxyl, Cyano, Nitro, Aryl und Benzyl, substituiert sein. Halogen kann dabei Fluor, Chlor, Brom oder Iod sein. Vorzugsweise handelt es sich bei dem Halogen um Fluor oder Chlor. Aryl ist vorzugsweise C₆-C₁₂-Aryl und umfasst beispielsweise Phenyl und Naphthyl. Bevorzugtes Aryl ist Phenyl. Gemäß einer bevorzugten Ausführungsform der offenbarten Verbindungen ist der Rest R¹¹ Methyl, Ethyl, Propyl, iso-Propyl, Butyl, Pentyl, Hexyl, Heptyl oder Octyl.

R¹², R¹³ und R¹⁴ sind jeweils unabhängig voneinander Wasserstoff, Halogen, geradkettiges oder verzweigtes C₁-C₁₂-Alkyl, C₃-C₈-Cycloalkyl, C₆-C₁₂-Aryl oder C₅-C₁₂-Heteroaryl. Halogen kann dabei Fluor, Chlor, Brom oder Iod sein. Vorzugsweise handelt es sich bei dem Halogen um Fluor oder Chlor. C₁-C₁₂-Alkyl ist beispielsweise Methyl, Ethyl, Propyl, iso-Propyl, Butyl, iso-Butyl, tert-Butyl, Pentyl, Hexyl, Heptyl, Octyl, Nonyl, Decyl, Undecyl oder Dodecyl. Vorzugsweise ist C₁-C₁₂-Alkyl Methyl, Ethyl, Propyl, iso-Propyl, Butyl, Pentyl, Hexyl, Heptyl oder Octyl. C₃-C₈-Cycloalkyl ist beispielsweise Cyclopropyl, Cyclobutyl, Cyclopentyl, Cyclohexyl, Cycloheptyl oder Cyclooctyl. Vorzugsweise ist C₃-C₈-Cycloalkyl Cyclohexyl. C₆-C₁₂-Aryl oder C₅-C₁₂-Heteroaryl sind beispielsweise Phenyl, Naphthyl, Furanyl, Imidazolyl, Pyrazolyl oder Pyridinyl. Vorzugsweise sind C₆-C₁₂-Aryl oder C₅-C₁₂-Heteroaryl Phenyl, Imidazolyl oder Pyridinyl. Die vorstehend genannten Substituenten C₁-C₁₂-Alkyl, C₃-C₈-Cycloalkyl, C₆-C₁₂-Aryl oder C₅-C₁₂-Heteroaryl können optional mit einem oder mehreren Substituenten, ausgewählt aus der Gruppe, bestehend aus Halogen, Hydroxyl, Cyano, Nitro, Aryl und Benzyl, substituiert sein. Halogen kann dabei Fluor, Chlor, Brom oder Iod sein. Vorzugsweise handelt es sich bei dem Halogen um Fluor oder Chlor. Aryl ist vorzugsweise C₆-C₁₂-Aryl und umfasst beispielsweise Phenyl und Naphthyl. Bevorzugtes Aryl ist Phenyl. Gemäß einer bevorzugten Ausführungsform der vorliegenden Erfindung sind die Reste R¹², R¹³ und R¹⁴ jeweils unabhängig voneinander aus Wasserstoff, Methyl, Ethyl, Propyl, iso-Propyl, Butyl, Pentyl, Hexyl, Heptyl oder Octyl ausgewählt.

R¹⁵ und R¹⁶ sind jeweils unabhängig voneinander geradkettiges oder verzweigtes C₁-C₁₂-Alkyl, C₃-C₈-Cycloalkyl, C₆-C₁₂-Aryl oder C₅-C₁₂-Heteroaryl. Geradkettiges oder verzweigtes C₁-C₁₂-Alkyl, C₃-C₈-Cycloalkyl, C₆-C₁₂-Aryl oder C₅-C₁₂-Heteroaryl sind wie vorstehend für R¹¹ definiert. Diese Reste können optional mit einem oder mehreren Substituenten, ausgewählt aus der Gruppe, bestehend aus Halogen, Cyano, Nitro, Aryl und Benzyl, substituiert sein, wobei Halogen und Aryl ebenfalls wie vorstehend bei R¹¹ definiert sind.

Gemäß einer bevorzugten Ausführungsform der offenbarten Verbindung ist Y eine Einfachbindung und die Reste R¹², R¹³ und R¹⁴ sind jeweils unabhängig voneinander aus Wasserstoff, Methyl, Ethyl, Propyl, iso-Propyl, Butyl, Pentyl, Hexyl, Heptyl oder Octyl ausgewählt. Gemäß einer weiteren bevorzugten Ausführungsform der offenbarten Verbindung ist X eine Einfachbindung und R¹¹ ist geradkettiges oder verzweigtes C₁-C₁₂-Alkyl, vorzugsweise Methyl, Ethyl, Propyl, iso-Propyl, Butyl, Pentyl, Hexyl, Heptyl oder Octyl. Gemäß einer weiteren bevorzugten Ausführungsform der offenbarten Verbindung ist Z eine Einfachbindung, n=3 und R¹⁵ Methyl, Ethyl oder iso-Propyl.

In erfindungsgemäßen Verbindungen ist einer der Reste R¹ bis R⁵ sowie einer der Reste R⁶ bis R¹⁰ -Y-C(R¹²)=C(R¹³)(R¹⁴), wobei Y jeweils eine Einfachbindung ist und die verbleibenden Reste von R¹ bis R¹⁰ Wasserstoff sind. Diese Verbindungen können beispielsweise symmetrisch sein, d.h. der ethylenisch ungesättigte Rest befindet sich in beiden Ringen jeweils in para-Position (allgemeine Formel (II)), ortho-Position (allgemeine Formel (III)) oder meta-Position (allgemeine Formel (IV)):

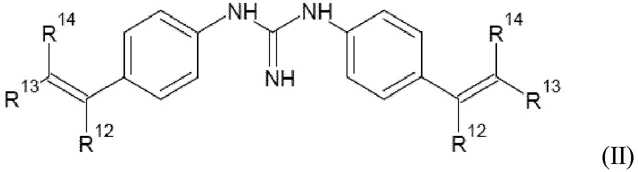

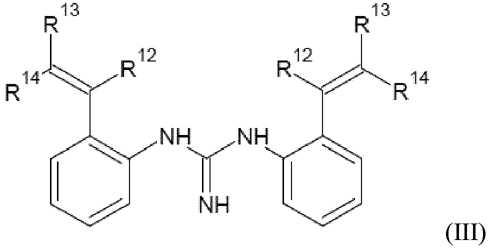

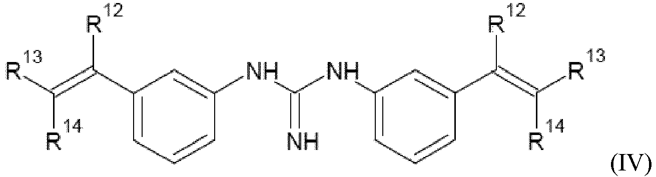

Es ist aber ebenso möglich, dass die erfindungsgemäßen Verbindungen unsymmetrisch sind, d.h. der ethylenisch ungesättigte Rest befindet sich in den beiden Ringen an unterschiedlichen Positionen (allgemeine Formeln (V) bis (VII)):

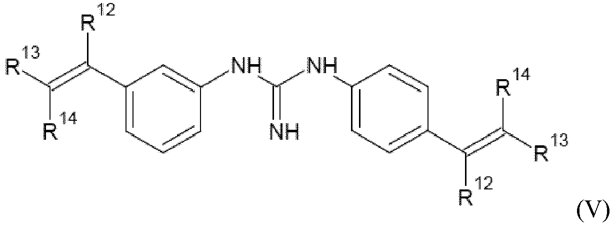

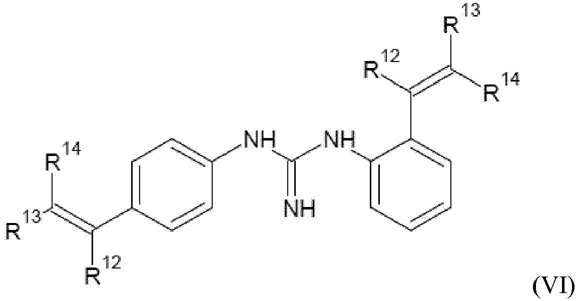

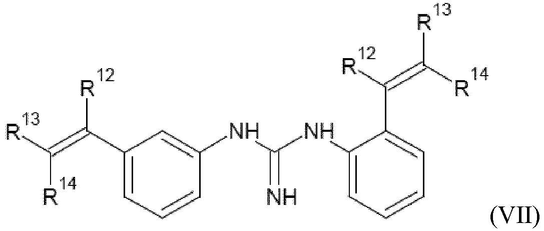

Die Reste R¹², R¹³ und R¹⁴ können jeweils gleich oder verschieden sein. Vorzugsweise sind die Reste R¹², R¹³ und R¹⁴ in beiden Substituenten jeweils identisch, wobei R¹² aus Wasserstoff oder Methyl ausgewählt ist und R¹³ und R¹⁴ Wasserstoff sind.

Gemäß einer besonders bevorzugten Ausführungsform weist die erfindungsgemäße Verbindung die allgemeine Formel (II), (III) oder (IV) auf. Dabei ist es insbesondere bevorzugt, dass R¹² aus Wasserstoff oder Methyl ausgewählt ist und R¹³ und R¹⁴ Wasserstoff sind (Formeln (II-1), (III-1), (III-2) und (IV-1)):

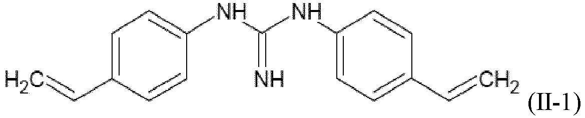

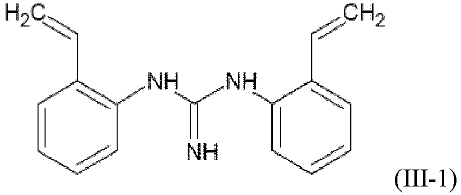

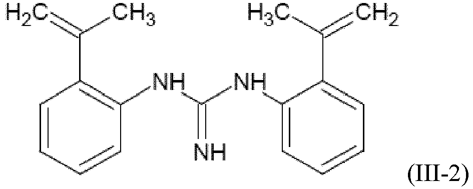

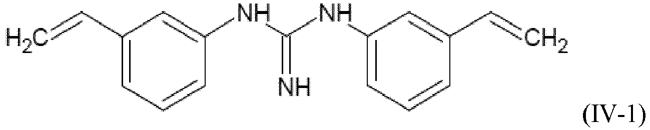

Die erfindungsgemäßen Verbindungen sind durch herkömmliche, dem Fachmann bekannte Verfahren zugänglich.

Die symmetrischen Verbindungen der allgemeinen Formeln (II) bis (IV) können beispielsweise ausgehend von einem geeigneten Vinylanilin hergestellt werden. Zur Herstellung der Verbindung (II-1) wird zum Beispiel 4-Vinylanilin mit Imidazol und Bromcyan unter Bildung des mit ethylenisch ungesättigten Gruppen substituierten Diphenylguanidins umgesetzt:

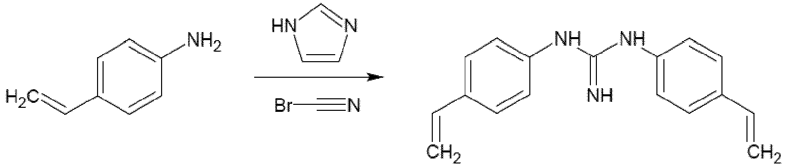

Das gleiche Reaktionsschema kann auch in entsprechender Weise zur Herstellung der ortho- und meta-substituierten Diphenylguanidine verwendet werden. Dazu wird nicht 4-Vinylanilin, sondern 2-Vinylanilin oder 3-Vinylanilin als Edukt verwendet.

Ferner kann die Verbindung gemäß Formel (II-1) auch durch die folgenden schematisch dargestellten Syntheseabläufe hergestellt werden:

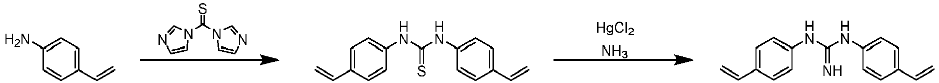

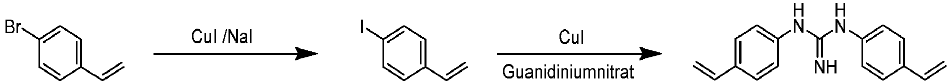

Die unsymmetrischen Verbindungen der allgemeinen Formeln (V) bis (VII) können in entsprechender, aber zweistufiger Weise hergestellt werden, was über eine geeignete Anpassung der Stöchiometrien möglich ist. Dabei wird in einem ersten Schritt der erste vinylsubstituierte Phenylring eingefügt, gefolgt von der separaten Einführung des zweiten vinylsubstituierten Phenylringes in einem zweiten Schritt:

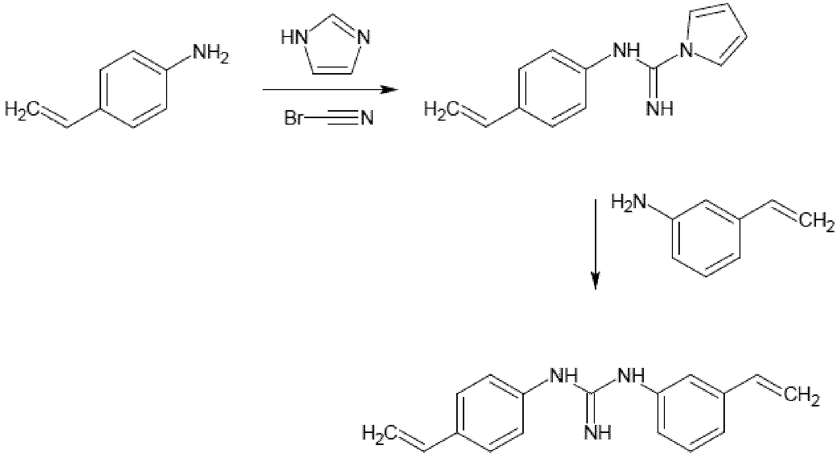

In entsprechender Weise können auch weitere unsymmetrisch substituierte Diphenylguanidine erhalten werden. Durch Wahl geeigneter, dem Fachmann bekannter Reagentien können auch die Reste R¹², R¹³ und R¹⁴ in geeigneter Weise variiert werden. Auf diese Weise sind ebenso monosubstituierte Derivate zugänglich, nämlich durch Verwendung von Anilin anstelle eines Vinylanilins in einem der beiden Reaktionsschritte.

Ferner können symmetrische und/oder unsymmetrische der Verbindungen der para,- ortho- und meta-substituierten Diphenylguanidine auf folgende Weise hergestellt werden:

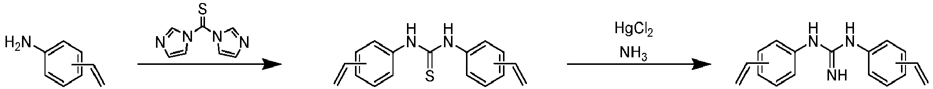

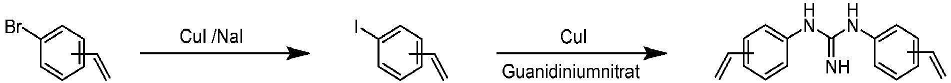

Die vorliegende Erfindung betrifft weiterhin eine Kautschukmischung, umfassend einen Kautschuk, einen Kieselsäure-basierten Füllstoff und/oder Ruß, und mindestens eine erfindungsgemäße Verbindung, d.h. mindestens ein modifiziertes Diphenylguanidin.

Erfindungsgemäß kann die Kautschukmischung jedweden geeigneten Kautschuk bzw. jedwede geeignete Kautschukkomponente enthalten. Bevorzugte Kautschuke sind insbesondere ein Ethylen-Propylen-Mischpolymerisat (EPM), ein Ethylen-Propylen-Dien-Mischpolymerisat (EPDM), (teil)hydrierter Nitrilkautschuk (HNBR), ChloroprenKautschuk (CR), Fluor-Kautschuk (FKM), Naturkautschuk (NR), Styrol-Butadien-Kautschuk (SBR), ein Butadien/Acrylsäure-C₁₋₄-Alkylester-Copolymerisat (ABR), Polyisopren (IR), ein Isobutyl-Isopren-Copolymerisat (IIR) oder Butadien-Kautschuk (BR). Diese können unverschnitten oder mit wenigsten einer weiteren Kautschukkomponente, insbesondere mit einem der vorgenannten Kautschuktypen, verschnitten eingesetzt werden, beispielsweise in Form eines EPM/EPDM- oder SBR/BR-Verschnittes. Gemäß einer besonders bevorzugten Ausführungsform umfasst der Kautschuk Ethylen-Propylen-Mischpolymerisat (EPM), Ethylen-Propylen-Dien-Mischpolymerisat (EPDM) oder einen Blend aus Ethylen-Propylen-Mischpolymerisat (EPM) und Ethylen-Propylen-Dien-Mischpolymerisat (EPDM) (auch als EPM/EPDM-Verschnitt bezeichnet).

Weiterhin kann erfindungsgemäß jedweder geeignete Kieselsäure-basierte Füllstoff verwendet werden. Bevorzugte Kieselsäure-basierte Füllstoffe sind Kieselsäure (insbesondere Fällungskieselsäure oder pyrogene Kieselsäure), synthetische Silikate (insbesondere Aluminiumsilikat oder Erdalkalisilikate) sowie natürliche Silikate (insbesondere Kaolin). Zusätzlich zu oder anstelle von den Kieselsäure-basierten Füllstoffen können jedwede geeignete Ruße als Füllstoffe verwendet werden. Bevorzugte Ruße sind nach dem Flammruß-, Furnace- oder Gasruß-Verfahren hergestellte Ruße, welche BET-Oberflächen (bestimmt nach ASTM D 3663-78) von 20 bis 200 m²/g aufweisen.

Die erfindungsgemäße Kautschukmischung enthält vorzugsweise 5 bis 200 phr ("parts per hundred rubber"), besonders bevorzugt 30 bis 150 phr an Kieselsäure-basierten Füllstoffen.

Der Gesamtanteil an zusätzlichen Füllstoffen, insbesondere Rußen in der Kautschukmischung beträgt typischerweise von 0 bis 160 phr, vorzugsweise 1 bis 100 phr, besonders bevorzugt 5 bis 80 phr. Sofern die Kautschukmischung Ruß und kieselsäurebasierende Füllstoffe enthält, so beträgt die Gesamtmenge dieser beiden Füllstoffarten vorzugsweise 20 bis 160 phr, besonders bevorzugt 25 bis 140 phr. In einer bevorzugten Ausführungsform ist das Gewichtsverhältnis zwischen Ruß-basierten Füllstoffen und Kieselsäure-basierten Füllstoffen kleiner als 1, vorzugsweise kleiner als 0,5.

Die erfindungsgemäße Kautschukmischung kann weiterhin jedwede geeignete Mischungsingredienzien enthalten. Diese umfassen vorzugweise wenigstens einen Vernetzer, wobei schwefelhaltige Silane, Schwefel-basierte oder peroxidische Vernetzer bevorzugt und Schwefel-basierte Vernetzer besonders bevorzugt sind. Weitere Mischungsingredienzien sind zumeist noch ein Verarbeitungshilfsmittel und/oder ein Weichmacher und/oder ein Alterungsschutzmittel sowie gegebenenfalls weitere Zusatzstoffe, beispielsweise Fasern und Farbpigmente. Diesbezüglich wird auf den allgemeinen Stand der Kautschukmischungstechnologie verwiesen.

Der Gehalt des erfindungsgemäßen, modifizierten Diphenylguanidins in der erfindungsgemäßen Kautschukmischung beträgt üblicherweise 0,01 bis 10 phr, vorzugsweise 0,03 bis 3 phr, besonders bevorzugt 0,1 bis 1 phr und ganz besonders bevorzugt 0,2 bis 0,5 phr.

Die erfindungsgemäße Kautschukmischung ist im Wesentlichen frei von unmodifiziertem Diphenylguanidin (DPG), da dieses bei der Vulkanisation zur Freisetzung von Anilin führen kann. Im Rahmen der vorliegenden Erfindung sind solche Kautschukmischungen als im Wesentlichen frei von unmodifiziertem Diphenylguanidin zu verstehen, die weniger als 0,1 phr Diphenylguanidin umfassen. Besonders bevorzugt enthält die Kautschukmischung kein unmodifiziertes Diphenylguanidin. Dies bedeutet erfindungsgemäß, dass in der Kautschukmischung weniger als 0,01 phr Diphenylguanidin enthalten sind.

Die vorliegende Erfindung betrifft ferner ein Verfahren zur Herstellung eines Kautschukvulkanisats, umfassend das Vulkanisieren der vorstehend beschriebenen erfindungsgemäßen Kautschukmischung bei einer Temperatur im Bereich von 100 bis 250°C, vorzugsweise bei einer Temperatur im Bereich von 130 bis 180°C, wobei gegebenenfalls ein Druck von 10 bis 200 bar angewendet werden kann.

Darüber hinaus betrifft die vorliegende Erfindung ein Vulkanisat, welches durch Vulkanisation der vorstehend beschriebenen, erfindungsgemäßen Kautschukmischung erhältlich ist.

Die vorliegende Erfindung betrifft ferner ein Kautschukprodukt, enthaltend das vorstehend beschriebene, erfindungsgemäße Vulkanisat. Dabei kann es sich um jedwedes geeignete Kautschukprodukt handelt. Vorzugsweise ist dieses ein Gummiartikel (insbesondere ein technischer Gummiartikel) oder ein Reifen. Weitere geeignete Kautschukprodukte stellen beispielsweise Reifenbauteile (insbesondere für Reifenlaufflächen, Subtreads, Karkassen, Seitenwände oder verstärkte Seitenwände für Notlaufreifen) oder technische Gummiartikel wie Dämpfungselemente, Walzenbeläge, Beläge von Förderbändern, Riemen, Dichtungen, Golfballkernen, Schuhsohlen, etc. dar. Besonders bevorzugt ist das Kautschukprodukt ein Reifen oder ein Antriebsriemen (insbesondere ein Flachriemen, Keilriemen, Keilrippenriemen, Zahnriemen, Kupplungsriemen oder Aufzugsriemen).

Die vorliegende Erfindung betrifft schließlich die Verwendung des erfindungsgemäßen modifizierten Diphenylguanidins als Sekundärbeschleuniger bei der Vulkanisation einer Kautschukmischung.

Im Folgenden soll die Erfindung anhand von zwei Synthesebeispielen der Verbindung gemäß Formel (II-1) verdeutlicht werden:

### 1. Beispiel

### 1.1 Synthese von 4-Iodstyrol:

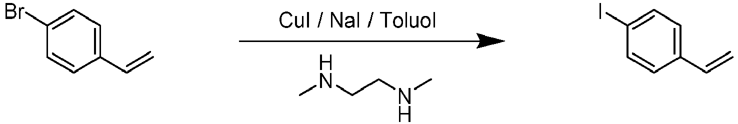

Unter Stickstoff wurden in 60 mL trockenem Toluol 7.00 g (1.0 Äq) 4-Bromstyrol gelöst und nacheinander 1.46 g (0.2 Äquivalente (Äq)) Kupferiodid, 1.35 g (0.4 Äq) N,N'-Diemthylethylendiamin sowie 11.46 g (2.0 Äq) Natriumiodid zugegeben. Es wurde für 22 Stunden bei 110 °C gerührt. Anschließend wurde auf Raumtemperatur (RT) gekühlt und das Lösungsmittel entfernt. Der Rückstand wurde in Dichlormethan (DCM) aufgenommen und mit verd. Ammoniaklösung, Wasser sowie gesättigter Kochsalzlösung ausgeschüttelt. Die organischen Phasen wurden vereinigt und über Natriumsulfat getrocknet. Die Salze wurden abfiltriert, das Lösungsmittel im Vakuum entfernt und der Rückstand säulenchromatographisch an Kieselgel aufgereinigt (Cyclohexan). Es wurde ein Gemisch aus 4-Iodstyrol und 4-Bromstyrol (70:30) erhalten, welches ohne weitere Aufreinigung in der nächsten Synthesestufe eingesetzt wurde. Weißer Feststoff; Ausbeute 3.15 g (36% d.Th., bezogen auf Iodstyrol).

¹H-NMR (*engl.* "nuclear magnetic resonance") (500 MHz, CDCl₃) δ = 7.70 (d, *J* = 8.4 Hz, 2H), 7.18 (d, *J* = 8.4 Hz, 2H), 6.75-6.65 (m, 1H), 5.82 (d, *J* = 17.6 Hz, 2H), 5.31 (d, *J* = 10.9 Hz, 2H).

### 1.2 Synthese von 1,3-Bis(4-vinylphenyl)guanidin:

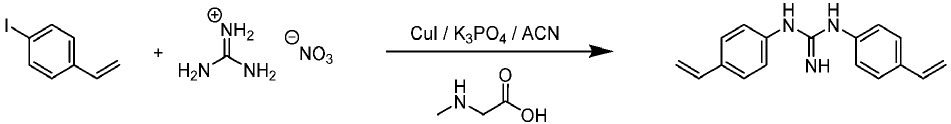

Unter Stickstoff wurden in 68.5 mL trockenem Acetonitril (ACN) 3.15 g (1.0 Äq) reines 4-Iodstyrol oder entsprechend eine Mischung aus 4-Iodstyrol und 4-Bromstyrrol vorgelegt, dass diese Mischung der reinen Menge von 3.15 g 4-Iodstyrol entspricht. Anschließend wurden 1.67 g (1.0 Äq) Guanindiniumnitrat, 17.44 g (6.0 Äq) Kaliumphosphat, 0.24 g (0.2 Äq) Sarcosin und 0.26 g (0.1 Äq) Kupferiodid zugefügt. Die Suspension wurde für 12 Stunden am Rückfluss erhitzt und danach auf RT abkühlen gelassen. Die anorganischen Anteile wurden per Filtration abgetrennt und das Lösungsmittel im Vakuum entfernt. Das Rohprodukt wurde säulenchromatographisch an Kieselgel aufgereinigt (Essigester/Cyclohexan 4:1 plus 1% TEA). Weißer Feststoff; Ausbeute 2.00 g (93 % d.Th.).

¹H-NMR (500 MHz, ACN-*d6*) δ = 7.39 (d, *J* = 8.5 Hz, 4H), 7.20 (d, *J* = 8.2 Hz, 4H), 6.72 (dd, *J=* 17.6, 10.9 Hz, 2H), 5.70 (dd, *J* = 17.7, 1.1 Hz, 2H), 5.15 (dd, *J* = 10.9 Hz, 2H).

¹³C-NMR (125 MHz, ACN-*d6*) δ = 148.1, 145.3, 131.1, 126.8, 121.0, 117.3, 111.0.

ESI-MS (Elektrosprayionisation Massenspektrometrie) [M+H]⁺ = 264.

### 2. Beispiel

### 2.1 Synthese von 1,3-Bis(4-vinylphenyl)thioharnstoff:

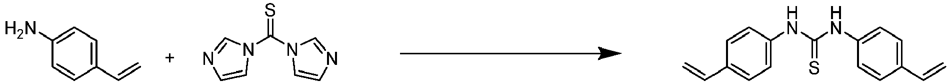

598 mg (1.0 Äq) 1,1'-Thiocarbonyldiimidazol wurden in 30 mL trockenem Tetrahydrofuran (THF) gelöst und 1.00 g 4-Vinylstyrol (1.0 Äq) langsam zugegeben. Es wurde 24 Stunden in der Siedehitze gerührt und im Anschluss das Lösungsmittel entfernt.

Der Rückstand wurde aus einem 2:1-Gemisch Cyclohexan/Essigester umkristallisiert. Es wurde ein brauner Feststoff erhalten, der in DCM aufgenommen und mit 1M HCl (1molare Salzsäure) sowie gesättigter NaHCOs-Lösung gewaschen wurde. Blass gelber Feststoff; Ausbeute 560 mg (60 % d.Th.).

¹H-NMR (500 MHz, DMSO-*d6*) δ = 9.87 (s, 2H), 7.49 (d, *J* = 8.7 Hz, 4H), 7.44 (d, *J* = 8.6 Hz, 4H), 6.71 (dd, *J* = 17.6, 10.9 Hz, 2H), 5.78 (dd, *J* = 17.6, 1.1 Hz, 2H), 5.22 (dd, *J* = 10.9, 1.0 Hz, 2H).

ESI-MS [M+H]⁺ = 281.

### 2.2 Synthese von 1,3-Bis(4-vinylphenyl)guanidin:

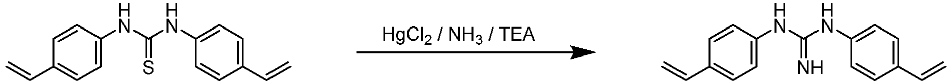

Unter Stickstoff wurden 250 mg (1.0 Äq) 1,3-Bis(4-vinylphenyl)thioharnstoff in 20 mL trockenem DMF gelöst und auf 0 °C gekühlt. Im Anschluss wurden 114 µL (2.0 Äq) wässrige Ammoniaklösung sowie 497 µL (4.0 Äq) Triethylamin zugtropft. Die gelbe Suspension wurde zwei Stunden lang bei 0 °C und über Nacht bei RT gerührt. Es erfolgte der Farbumschlag nach schwarz (HgS-Bildung). Die entstandenen Feststoffe wurden abfiltriert und das Filtrat zur Trockne eingeengt. Weißer Feststoff; Ausbeute 759 mg (85 % d. Th.).

¹H-NMR (500 MHz, ACN-*d6*) δ = 7.39 (d, *J* = 8.5 Hz, 4H), 7.20 (d, *J* = 8.2 Hz, 4H), 6.72 (dd, *J* = 17.6, 10.9 Hz, 2H), 5.70 (dd, *J* = 17.7, 1.1 Hz, 2H), 5.15 (dd, *J* = 10.9 Hz, 2H).

¹³C-NMR (125 MHz, ACN-*d6*) δ = 148.1, 145.3, 131.1, 126.8, 121.0, 117.3, 111.0.

ESI-MS [M+H]⁺ = 264.

## Patentansprüche

1. Verbindung mit der allgemeinen Formel (I) 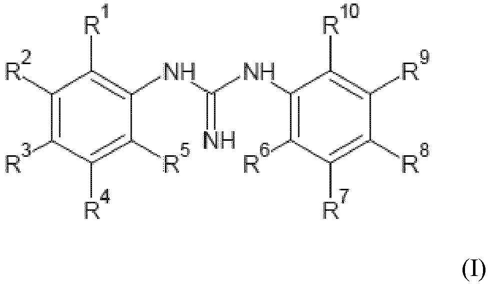
wobei die Reste R¹ bis R¹⁰ unabhängig voneinander aus der Gruppe, bestehend aus Wasserstoff, und -Y-C(R¹²)=C(R¹³)(R¹⁴) ausgewählt sind,
wobei
R¹², R¹³ und R¹⁴ jeweils unabhängig voneinander Wasserstoff, Halogen, geradkettiges oder verzweigtes C₁-C₁₂-Alkyl, C₃-C₈-Cycloalkyl, C₆-C₁₂-Aryl oder C₅-C₁₂-Heteroaryl ist, wobei das geradkettige oder verzweigte C₁-C₁₂-Alkyl, C₃-C₈-Cycloalkyl, C₆-C₁₂-Aryl und C₅-C₁₂-Heteroaryl optional mit einem oder mehreren Substituenten, ausgewählt aus der Gruppe, bestehend aus Halogen, Hydroxyl, Cyano, Nitro, Aryl und Benzyl, substituiert sein kann,
**dadurch gekennzeichnet, dass** einer der Reste R¹ bis R⁵ sowie einer der Reste R⁶ bis R¹⁰ -Y-C(R¹²)=C(R¹³)(R¹⁴) ist, dabei Y jeweils eine Einfachbindung ist und die verbleibenden Reste von R¹ bis R¹⁰ Wasserstoff sind.

2. Verbindung nach Anspruch 1, wobei die Reste R¹², R¹³ und R¹⁴ jeweils unabhängig voneinander aus Wasserstoff, Methyl, Ethyl, Propyl, iso-Propyl, Butyl, Pentyl, Hexyl, Heptyl oder Octyl ausgewählt sind.

3. Verbindung nach Anspruch 1 oder 2 mit der allgemeinen Formel (II):

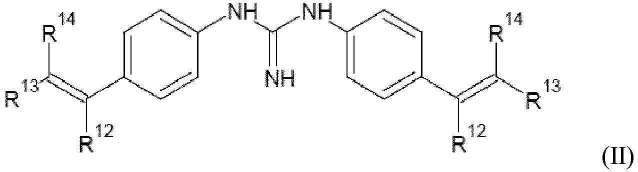

4. Verbindung nach einem der Ansprüche 1 bis 3, wobei die Reste R¹², R¹³ und R¹⁴ in beiden Substituenten jeweils identisch sind und R¹² aus Wasserstoff oder Methyl ausgewählt ist und R¹³ und R¹⁴ Wasserstoff sind.

5. Kautschukmischung, umfassend einen Kautschuk, einen Kieselsäure-basierten Füllstoff und/oder Ruß, und mindestens eine Verbindung gemäß einem der Ansprüche 1 bis 4.

6. Kautschukmischung nach Anspruch 5, wobei der Gehalt der mindestens einen Verbindung gemäß einem der Ansprüche 1 bis 7 0,01 bis 10 phr beträgt.

7. Kautschukmischung nach Anspruch 5 oder 6, welche kein unmodifiziertes Diphenylguanidin enthält.

8. Verfahren zur Herstellung eines Kautschukvulkanisats, umfassend das Vulkanisieren der Kautschukmischung nach einem der Ansprüche 5 bis 7 bei einer Temperatur im Bereich von 100 bis 250°C.

9. Vulkanisat, erhältlich durch Vulkanisation der Kautschukmischung nach einem der Ansprüche 5 bis 7.

10. Kautschukprodukt, vorzugsweise ein Gummiartikel oder Reifen, enthaltend das Vulkanisat nach Anspruch 9.

11. Verwendung der Verbindung nach einem der Ansprüche 1 bis 4 als Sekundärbeschleuniger bei der Vulkanisation einer Kautschukmischung.

## Claims

1. Compound having the general formula (I) 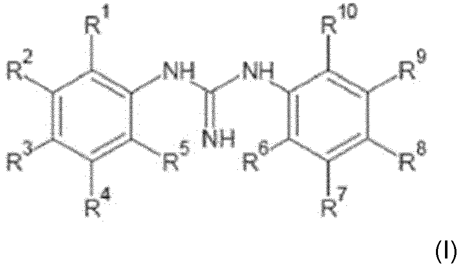
where the radicals R¹ to R¹⁰ are each independently selected from the group consisting of hydrogen and -Y-C(R¹²)=C(R¹³)(R¹⁴),
where
R¹², R¹³ and R¹⁴ are each independently hydrogen, halogen, straight-chain or branched C₁-C₁₂ alkyl, C₃-C₈ cycloalkyl, C₆-C₁₂ aryl or C₅-C₁₂ heteroaryl, wherein the straight-chain or branched C₁-C₁₂ alkyl, C₃-C₈ cycloalkyl, C₆-C₁₂ aryl or C₅-C₁₂ heteroaryl may optionally be substituted with one or more substituents selected from the group consisting of halogen, hydroxyl, cyano, nitro, aryl and benzyl,
**characterized in that** one of radicals R¹ to R⁵ and also one of radicals R⁶ to R¹⁰ is -Y-C(R¹²)=C(R¹³)(R¹⁴), where Y is in both cases a single bond, and the remaining radicals R¹ to R¹⁰ are hydrogen.

2. Compound according to Claim 1, wherein the radicals R¹², R¹³ and R¹⁴ are each independently selected from hydrogen, methyl, ethyl, propyl, isopropyl, butyl, pentyl, hexyl, heptyl or octyl.

3. Compound according to Claim 1 or 2 having the general formula (II):

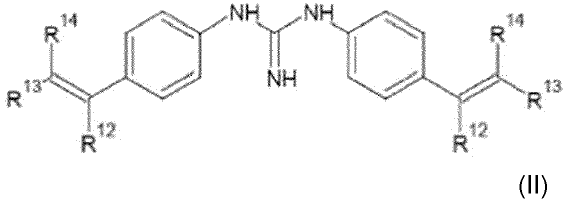

4. Compound according to any of Claims 1 to 3, where the radicals R¹², R¹³ and R¹⁴ are in both substituents identical and R¹² is selected from hydrogen or methyl and R¹³ and R¹⁴ are hydrogen.

5. Rubber mixture, comprising a rubber, a silica-based filler and carbon black, and at least one compound according to any of Claims 1 to 4.

6. Rubber mixture according to Claim 5, wherein the content of the at least one compound according to any of Claims 1 to 7 is 0.01 to 10 phr.

7. Rubber mixture according to Claim 5 or 6, which contains no unmodified diphenylguanidine.

8. Process for producing a rubber vulcanizate, comprising the vulcanization of the rubber mixture according to any of Claims 5 to 7 at a temperature in the range from 100 to 250°C.

9. Vulcanizate obtainable by vulcanizing the rubber mixture according to any of Claims 5 to 7.

10. Rubber product, preferably a rubber article or tyre, comprising the vulcanizate according to Claim 9.

11. Use of the compound according to any of Claims 1 to 4 as a secondary accelerator in the vulcanization of a rubber mixture.

## Revendications

1. Composé doté de la formule générale (I) 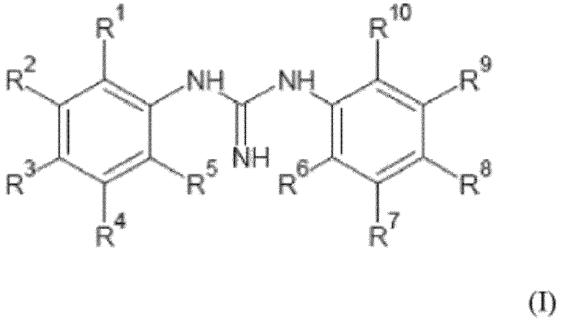 les radicaux R¹ à R¹⁰ étant choisis indépendamment les uns des autres dans le groupe constitué par hydrogène, et -Y-C(R¹²)=C(R¹³)(R¹⁴), R¹², R¹³ et R¹⁴ étant chacun indépendamment les uns des autres hydrogène, halogène, C₁-C₁₂ alkyle linéaire ou ramifié, C₃-C₈ cycloalkyle, C₆-C₁₂ aryle ou C₅-C₁₂ hétéroaryle, le C₁-C₁₂ alkyle linéaire ou ramifié, C₃-C₈ cycloalkyle, C₆-C₁₂ aryle ou C₅-C₁₂ hétéroaryle pouvant éventuellement être substitué par un ou plusieurs substituants choisis dans le groupe constitué par halogène, hydroxyle, cyano, nitro, aryle et benzyle, **caractérisé en ce que** l'un des radicaux R¹ à R⁵ et également l'un des radicaux R⁶ à R¹⁰ est -Y-C(R¹²)=C(R¹³)(R¹⁴), Y étant à chaque fois une simple liaison et les radicaux restants de R¹ à R¹⁰ étant hydrogène.

2. Composé selon la revendication 1, les radicaux R¹², R¹³ et R¹⁴ étant chacun indépendamment les uns des autres choisis parmi hydrogène, méthyle, éthyle, propyle, iso-propyle, butyle, pentyle, hexyle, heptyle ou octyle.

3. Composé selon la revendication 1 ou 2 doté de la formule générale (II) :

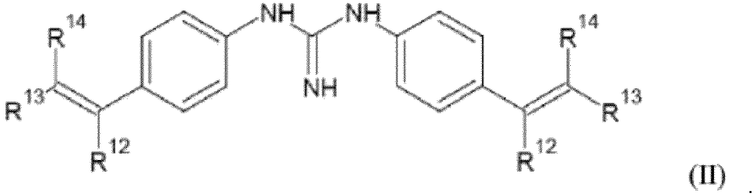

4. Composé selon l'une quelconque des revendications 1 à 3, les radicaux R¹², R¹³ et R¹⁴ étant à chaque fois identiques dans les deux substituants et R¹² étant choisi parmi hydrogène ou méthyle et R¹³ et R¹⁴ étant hydrogène.

5. Mélange de caoutchouc, comprenant un caoutchouc, une charge à base de silice et/ou de la suie, et au moins un composé selon l'une quelconque des revendications 1 à 4.

6. Mélange de caoutchouc selon la revendication 5, la teneur de l'au moins un composé selon l'une quelconque des revendications 1 à 7 étant de 0,01 à 10 phr.

7. Mélange de caoutchouc selon la revendication 5 ou 6, qui ne contient aucune diphénylguanidine non modifiée.

8. Procédé de préparation d'un vulcanisat de caoutchouc, comprenant la vulcanisation du mélange de caoutchouc selon l'une quelconque des revendications 5 à 7 à une température dans la plage de 100 à 250 °C.

9. Vulcanisat pouvant être obtenu par vulcanisation du mélange de caoutchouc selon l'une quelconque des revendications 5 à 7.

10. Produit de caoutchouc, de préférence article en caoutchouc ou pneu, contenant le vulcanisat selon la revendication 9.

11. Utilisation du composé selon l'une quelconque des revendications 1 à 4 en tant qu'accélérateur secondaire lors de la vulcanisation d'un mélange de caoutchouc.
